# EUROPEAN PATENT APPLICATION

(11) **EP 4 591 727 A1**
(43) Date of publication of application: **30.07.2025**
(21) Application number: 23866925.3
(22) Date of filing: 28.04.2023
(51) Int. Cl.: A24F 40/40, A24F 40/48, A24F 40/10

(54) **ATOMIZER AND ELECTRONIC ATOMIZATION APPARATUS**

(30) Priority: 20.09.2022 CN 202211146654
(71) Applicant: Hainan Moore Brothers Technology Co., Ltd., Hainan 571900 (CN)
(72) Inventor: YANG, Hao, Hainan 571900 (CN); XU, Chaoping, Hainan 571900 (CN); REN, Sanbing, Hainan 571900 (CN); LEI, Guilin, Hainan 571900 (CN)
(74) Representative: Herrero & Asociados, S.L.
(86) International application number: PCT/CN2023/091835
(87) International publication number: WO 2024/060628

(57) **Abstract**

An atomizer (100) and an electronic atomization apparatus (300). The atomizer (100) comprises a liquid storage cavity (13) and a ventilation channel (3); the liquid storage cavity (13) is used for storing an aerosol generating matrix; a first end (31) of the ventilation channel (3) is communicated with the liquid storage cavity (13); the ventilation channel (3) comprises a shrinkage portion and/or an expansion portion, so as to prevent the aerosol generating matrix from flowing out of the ventilation channel (3) through a second end (32) of the ventilation channel (3). By means of the described configuration, the structure of the shrinkage portion and/or the expansion portion of the ventilation channel enables a capillary force direction in the ventilation channel to be changed, so that the aerosol generating matrix in the ventilation channel is effectively prevented from flowing out of the ventilation channel through the second end, the problem in the prior art of easy leakage of the aerosol generating matrix in the ventilation channel is solved, and the performance of the atomizer is improved.

## Description

### TECHNICAL FIELD

This application relates to the field of atomizer technologies, and in particular, to an atomizer and an electronic atomization apparatus.

### BACKGROUND

An electronic atomization apparatus generally includes an atomizer and a power supply assembly, and the power supply assembly is electrically connected to the atomizer to provide energy to the atomizer. The atomizer includes a liquid storage cavity. An aerosol-forming medium is stored in the liquid storage cavity. The atomizer is configured to heat and atomize the aerosol-forming medium to generate an aerosol.

To balance the air pressure in the liquid storage cavity, the atomizer is generally provided with a ventilation channel, and the aerosol-forming medium in the liquid storage cavity enters the ventilation channel under an action of capillary force. In the related art, when the external temperature or pressure changes, the ventilation channel is easily filled with liquid, causing the aerosol-forming medium in the ventilation channel to flow out of the ventilation channel.

### SUMMARY

This application mainly provides an atomizer and an electronic atomization apparatus, to resolve a related art problem that an aerosol-forming medium in a ventilation channel is prone to leakage.

To resolve the foregoing technical problem, a technical solution used in this application is: to provide an atomizer, including:
a liquid storage cavity, configured to store an aerosol-forming medium; and
a ventilation channel, where a first end of the ventilation channel is communicated with the liquid storage cavity; and the ventilation channel includes a narrowed portion and/or an expanded portion configured to prevent the aerosol-forming medium from flowing out of the ventilation channel from a second end of the ventilation channel.

In some embodiments, the ventilation channel includes a main channel section and at least one narrowed section that are communicated with each other, where each narrowed section includes the narrowed portion and/or the expanded portion, or the each narrowed section and the main channel section jointly form the narrowed portion and/or the expanded portion; and/or
the ventilation channel includes the main channel section and at least one expanded section that are communicated with each other, where each expanded section includes the narrowed portion and/or the expanded portion, or the each narrowed section and the main channel section jointly form the narrowed portion and/or the expanded portion.

In some embodiments, the ventilation channel includes the at least one narrowed section, an equivalent diameter of the each narrowed section gradually decreases along a direction from the first end to the second end of the ventilation channel to form a first narrowed portion, and then gradually increases to form a first expanded portion, or the equivalent diameter of the each narrowed section remains unchanged along the direction from the first end to the second end of the ventilation channel, and the equivalent diameter of the each narrowed section is shorter than an equivalent diameter of the main channel section; or
the ventilation channel includes the at least one expanded section, an equivalent diameter of the each expanded section gradually increases along an extension direction from the first end to the second end of the ventilation channel to form a second expanded portion, and then gradually decreases to form a second narrowed portion, or the equivalent diameter of the each expanded section remains unchanged along the direction from the first end to the second end of the ventilation channel, and the equivalent diameter of the each expanded section is longer than the equivalent diameter of the main channel section.

In some embodiments, the each narrowed section further includes a first uniform connection portion communicating the first narrowed portion and the first expanded portion, and an equivalent diameter of the first uniform connection portion remains unchanged along the direction from the first end to the second end of the ventilation channel; or
the ventilation channel includes the at least one expanded section, the each expanded section further includes a second uniform connection portion communicating the second narrowed portion and the second expanded portion, and an equivalent diameter of the second uniform connection portion remains unchanged along the direction from the first end to the second end of the ventilation channel.

In some embodiments, the narrowed portion or the expanded portion transitions in a curved line form along a direction from the first end to the second end of the ventilation channel; or
the narrowed portion or the expanded portion transitions in a straight line form along the direction from the first end to the second end of the ventilation channel; or
the narrowed portion or the expanded portion transitions in a stepwise form along a direction from the first end to the second end of the ventilation channel.

In some embodiments, the ventilation channel includes the main channel section and the at least one narrowed section that are communicated with each other, where the maximum equivalent diameter of the each narrowed section is 0.2 mm to 1 mm.

In some embodiments, the ventilation channel includes the main channel section and the at least one narrowed section that are communicated with each other, where the minimum equivalent diameter of the each narrowed section is shorter than the maximum equivalent diameter of the each narrowed section; and the ratio of the maximum equivalent diameter of the each narrowed section to the minimum equivalent diameter of the each narrowed section is M, where 1<M<4.

In some embodiments, a side wall of the ventilation channel has a lyophilic property.

In some embodiments, a material of the side wall of the ventilation channel is a lipophilic material; or the side wall of the ventilation channel is provided with a lipophilic material coating; or
the side wall of the ventilation channel has a lipophilic micro-nano structure.

In some embodiments, the atomizer includes:
a housing, provided with an air outlet tube and an accommodating cavity; and
a mounting base, disposed in the accommodating cavity, where the mounting base and the housing jointly form the liquid storage cavity; the mounting base is internally provided with an atomization cavity, and the atomization cavity is communicated with the air outlet tube and external atmosphere;
the ventilation channel is provided in the mounting base; and the second end of the ventilation channel is communicated with the atomization cavity or the external atmosphere.

In some embodiments, the ventilation channel is in a shape of a straight line structure; and
the first end of the ventilation channel is directly communicated with the liquid storage cavity, and the second end is communicated with the atomization cavity.

In some embodiments, the atomizer further includes a sealing member, an outer side surface of the mounting base is provided with a micro groove, and the sealing member covers the micro groove to form the ventilation channel; or
the atomizer further includes a ventilation member, the ventilation channel is provided in the ventilation member, and the ventilation member is disposed on the mounting base.

In some embodiments, the atomizer includes the ventilation member, and the ventilation member is a hollow tube used as the ventilation channel; a part of a wall of the hollow tube curves inward to form a narrowed section, and the narrowed section includes a first narrowed portion and a first expanded portion, and/or a part of the wall of the hollow tube is expanded outward to form an expanded section, and the expanded section includes a second narrowed portion and a second expanded portion.

In some embodiments, a material of the sealing member and/or a material of the mounting base are/is a lipophilic material; or a material of the ventilation member is a lipophilic material.

In some embodiments, the atomizer includes only one ventilation channel.

In some embodiments, the atomizer further includes an atomization core, the atomization core is disposed in the mounting base and is fluidly communicated with the liquid storage cavity, and the atomization core includes a porous liquid-guiding substrate and a heating element.

To resolve the foregoing technical problem, another technical solution used in this application is: to provide an electronic atomization apparatus, including:
an atomizer, where the atomizer is any one of the atomizers mentioned above; and
a power supply assembly, electrically connected to the atomizer, and configured to provide energy to the atomizer.

This application has the following technical effects. Different from the related art, this application provides an atomizer and an electronic atomization apparatus. The atomizer includes a liquid storage cavity and a ventilation channel. The liquid storage cavity is configured to store an aerosol-forming medium. A first end of the ventilation channel is communicated with the liquid storage cavity. The ventilation channel includes a narrowed portion and/or an expanded portion configured to prevent the aerosol-forming medium from flowing out of the ventilation channel from a second end of the ventilation channel. Based on the foregoing configuration, the structure of the narrowed portion and/or the expanded portion of the ventilation channel changes the capillary force direction in the ventilation channel, effectively preventing the aerosol-forming medium in the ventilation channel from flowing out of the ventilation channel from the second end, thereby resolving a related art problem that the aerosol-forming medium in the ventilation channel is prone to leakage, and improving the performance of the atomizer.

### BRIEF DESCRIPTION OF THE DRAWINGS

To describe the technical solutions in embodiments of this application or the related art more clearly, the following briefly describes the accompanying drawings required for describing the embodiments or the related art. Apparently, the accompanying drawings in the following descriptions show merely some embodiments of this application, and a person of ordinary skill in the art may still derive other accompanying drawings from the accompanying drawings without creative efforts.
FIG. 1 is a schematic diagram of a structure of an electronic atomization apparatus according to this application;
FIG. 2 a schematic diagram of a structure of a first embodiment of an atomizer of the electronic atomization apparatus provided in FIG. 1;
FIG. 3 is a schematic diagram of a structure of a first embodiment of a mounting base of the atomizer provided in FIG. 2;
FIG. 4 is a schematic diagram of a structure of a first implementation of a ventilation channel of the mounting base provided in FIG. 3;
FIG. 5 is a schematic diagram of a structure of a second implementation of a ventilation channel of the mounting base provided in FIG. 3;
FIG. 6 is a schematic diagram of a structure of a third implementation of a ventilation channel of the mounting base provided in FIG. 3;
FIG. 7 is a schematic diagram of a structure of a fourth implementation of a ventilation channel of the mounting base provided in FIG. 3;
FIG. 8 is a schematic diagram of a structure of a fifth implementation of a ventilation channel of the mounting base provided in FIG. 3;
FIG. 9 is a schematic diagram of a structure of a sixth implementation of a ventilation channel of the mounting base provided in FIG. 3;
FIG. 10 is a schematic diagram of a structure of a seventh implementation of a ventilation channel of the mounting base provided in FIG. 3;
FIG. 11 is a schematic diagram of a structure of an eighth implementation of a ventilation channel of the mounting base provided in FIG. 3;
FIG. 12 is a schematic diagram of a structure of the ventilation channel shown in FIG. 4 during a liquid storage process;
FIG. 13 is a schematic diagram of a structure of the ventilation channel shown in FIG. 4 during a ventilation process;
FIG. 14 is a schematic diagram of a structure of a second embodiment of a mounting base of the atomizer provided in FIG. 2;
FIG. 15 is a schematic diagram of a structure of a first implementation of a ventilation channel of the mounting base provided in FIG. 14;
FIG. 16 is a schematic diagram of a structure of a second implementation of a ventilation channel of the mounting base provided in FIG. 14;
FIG. 17 is a schematic diagram of a structure of a third implementation of a ventilation channel of the mounting base provided in FIG. 14;
FIG. 18 is a schematic diagram of a structure of a fourth implementation of a ventilation channel of the mounting base provided in FIG. 14;
FIG. 19 is a schematic diagram of a structure of the ventilation channel shown in FIG. 17 during a liquid storage process;
FIG. 20 is a schematic diagram of a structure of a third embodiment of a mounting base of the atomizer provided in FIG. 2;
FIG. 21 is a schematic diagram of simulation comparison between a liquid storage process of a third embodiment of an atomizer and a liquid storage process of a comparative example of an atomizer; and
FIG. 22 is a schematic diagram of simulation comparison between a ventilation process of a third embodiment of an atomizer and a ventilation process of a comparative example of an atomizer.

### DETAILED DESCRIPTION

The technical solutions in embodiments of this application are clearly and completely described below with reference to the accompanying drawings in embodiments of this application. Apparently, the described embodiments are merely some rather than all of embodiments of this application. All other embodiments obtained by a person of ordinary skill in the art based on embodiments of this application without creative efforts shall fall within the protection scope of this application.

The terms "first", "second", and "third" in embodiments of this application are merely intended for a purpose of description, and shall not be understood as an indication or implication of relative importance or implicit indication of the quantity of indicated technical features. Therefore, features defining "first", "second", and "third" may explicitly or implicitly include at least one of the features. In descriptions of this application, "a plurality of" means at least two, such as two and three unless it is specifically defined otherwise. In addition, the terms "include", "have", and any variant thereof are intended to cover a non-exclusive inclusion. For example, a process, method, system, product, or device that includes a series of operations or units is not limited to the listed operations or units, and instead, further optionally includes an operation or a unit that is not listed, or further optionally includes another operation or unit that is intrinsic to the process, method, product, or device.

"Embodiment" mentioned in the specification means that particular features, structures, or characteristics described with reference to an embodiment may be included in at least one embodiment of this application. The term appearing at different positions of this specification may not refer to the same embodiment or an independent or alternative embodiment that is mutually exclusive with another embodiment. A person of ordinary skill in the art explicitly or implicitly understands that embodiments described in the specification may be combined with other embodiments.

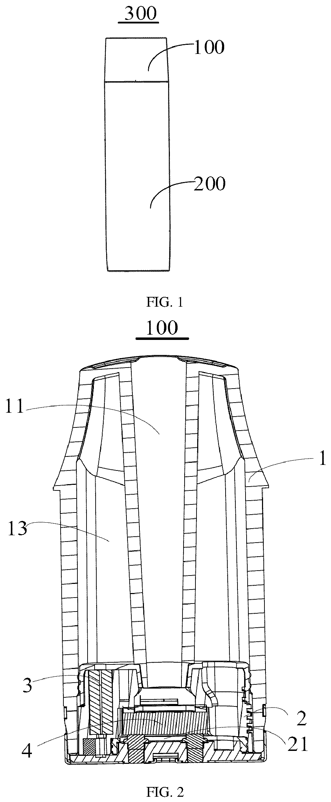

FIG. 1 is a schematic diagram of a structure of an electronic atomization apparatus according to this application.

Referring to FIG. 1, this application provides an electronic atomization apparatus 300, and the electronic atomization apparatus 300 may be configured to atomize an aerosol-forming medium. The electronic atomization apparatus 300 includes an atomizer 100 and a power supply assembly 200 that are electrically connected to each other.

The atomizer 100 is configured to store an aerosol-forming medium and atomize the aerosol-forming medium to form an aerosol that may be inhaled by a user. The atomizer 100 may be used in different fields such as medical care, cosmetology, and recreational vaping, etc. In some embodiments, the atomizer 100 may be disposed in an electronic aerosol atomization apparatus to atomize an aerosol-forming medium and generate an aerosol for inhalation by an inhaler. The following embodiments are described by using an example in which the atomizer is used in the field of recreational vaping.

For a structure and functions of the atomizer 100, reference may be made to the structure and functions of the atomizer 100 involved in the following embodiments, same or similar technical effects may also be implemented, and details are not described herein again.

The power supply assembly 200 may include a battery (not shown in the figure) and a controller (not shown in the figure). The battery is configured to supply electric energy for operation of the atomizer 100, to enable the atomizer 100 to heat and atomize the aerosol-forming medium to form an aerosol. The controller is configured to control the operation of the atomizer 100. The power supply assembly 200 may include other components such as a battery holder (not shown in the figure) and an airflow sensor (not shown in the figure).

FIG. 2 is a schematic diagram of a structure of a first embodiment of an atomizer of the electronic atomization apparatus provided in FIG. 1.

Referring to FIG. 2, the atomizer 100 of the electronic atomization apparatus 300 provided in this application may include a housing 1, a mounting base 2, and a ventilation channel 3. The housing 1 is provided with an air outlet tube 11 and an accommodating cavity (not shown in the figure), the mounting base 2 is disposed in the accommodating cavity, the mounting base 2 and the housing 1 jointly form a liquid storage cavity 13, and an aerosol-forming medium is stored in the liquid storage cavity 13. The mounting base 2 is internally provided with an atomization cavity 21 inside, and the atomization cavity 21 is communicated with the air outlet tube 11 and the external atmosphere. The ventilation channel 3 is defined in the mounting base 2, a first end 31 of the ventilation channel 3 is communicated with the liquid storage cavity 13, and a second end 32 of the ventilation channel 3 is communicated with the external atmosphere or the atomization cavity 21.

The side wall of the ventilation channel 3 has a lyophilic property, such as hydrophilicity or lipophilicity. In this embodiment, the aerosol-forming medium is an oily liquid, and the side wall of the ventilation channel 3 has a lipophilic property. In some implementations, the ventilation channel 3 may be made of a lipophilic material, for example, the side wall of the ventilation channel 3 may be made of a PCTG material. In some implementations, the side wall of the ventilation channel 3 may be coated with a lipophilic material coating, so that the side wall of the ventilation channel 3 has a lipophilic property. In some implementations, the side wall of the ventilation channel 3 may be configured to have a lipophilic micro-nano structure, so that the side wall of the ventilation channel 3 has a lipophilic property. Because the side wall of the ventilation channel 3 has a lipophilic property, when the aerosol-forming medium enters the ventilation channel 3, a capillary force direction A points from the first end 31 to the second end 32 of the ventilation channel 3.

In this embodiment, the ventilation channel 3 may include a narrowed portion and/or an expanded portion, to prevent the aerosol-forming medium from flowing out of the ventilation channel 3 from the second end 32. The narrowed portion in this application refers to a portion of the ventilation channel 3 where the equivalent diameter changes from long to short, and the expanded portion refers to a portion of the ventilation channel 3 where the equivalent diameter changes from short to long. This change trend may be abrupt or gradual. The gradual change may follow a straight line path, a curved line path, or a stepwise line path.

It may be understood that, in this embodiment, the narrowed portion and/or the expanded portion are/is provided in the ventilation channel 3 communicating the liquid storage cavity 13 with the external atmosphere/atomization cavity 21, so that the equivalent diameter of the ventilation channel 3 changes at the narrowed portion and/or the expanded portion. As a result, the capillary force direction A in the ventilation channel 3 also changes at the narrowed portion and/or the expanded portion, so as to prevent a problem that the ventilation channel 3 is easily filled with liquid due to the continuous entry of the aerosol-forming medium into the ventilation channel 3 from the liquid storage cavity 13 because the capillary force direction A in the ventilation channel 3 always points from the liquid storage cavity 13 to the external atmosphere/atomization cavity 21, specifically, the capillary force direction A always points from the first end 31 to the second end 32 and remains unchanged, due to the lipophilic property of the side wall of the ventilation channel 3. In this way, a problem of liquid leakage from the atomizer 100 caused by the aerosol-forming medium in the ventilation channel 3 flowing out of the ventilation channel 3 from the second end 32 is prevented, a related art problem that the aerosol-forming medium in the ventilation channel 3 is prone to leakage is resolved, thereby improving the atomization performance of the atomizer 100.

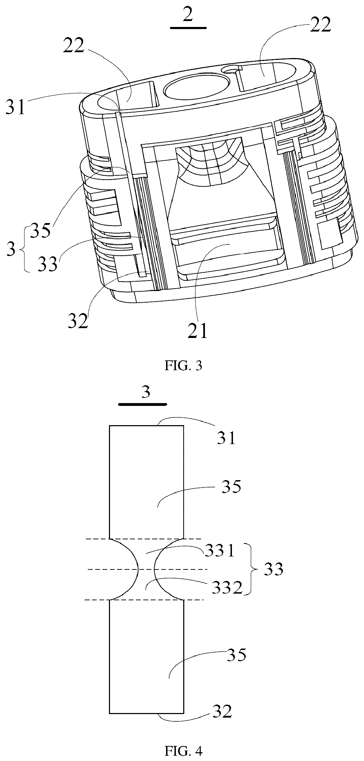

In some implementations, the atomizer 100 may include an atomization core 4. The atomization core 4 is disposed in the mounting base 2. The atomization core 4 and the mounting base 2 jointly form the atomization cavity 21. An atomization process takes place in the atomization cavity 21. The mounting base 2 is provided with a liquid inlet hole 22 (as shown in FIG. 3), the atomization core 4 is fluidly communicated with the liquid storage cavity 13, and the aerosol-forming medium stored in the liquid storage cavity 13 is absorbed by the atomization core 4 via the liquid inlet hole 22 of the mounting base 2. The atomization core 4 may include a porous liquid-guiding substrate (not shown in the figure) and a heating element (not shown in the figure). The porous liquid-guiding substrate may be made of a porous material such as porous ceramics or porous glass, or may be made of a dense ceramic material with a porous structure formed by creating openings. The heating element may be any metal heating structure, for example, a heating film, a heating wire, or a heating net. The heating element is configured to generate heat when being energized, so as to heat and atomize the aerosol-forming medium in the atomization core 4 to generate the aerosol. The atomization cavity 21 is communicated with the air outlet tube 11, and the aerosol generated by atomization flows to the air outlet tube 11 through the atomization cavity 21 and is finally inhaled by a user.

In this embodiment, the atomizer 100 may include one or more ventilation channels 3. It may be understood that when the atomizer 100 includes only one ventilation channel 3, a related art problem of aerosol-forming medium leakage in the atomizer 100 that includes two or more ventilation channels 3 may be resolved. The related art problem is that, when the pressure in the plurality of ventilation channels 3 is asymmetric, for example, when the atomizer 100 is tilted, one ventilation channel 3 ventilates while another ventilation channel 3 absorbs liquid, the aerosol-forming medium in the ventilation channel 3 on the liquid absorption side is prone to flowing out of the ventilation channel 3,. In other implementations, the atomizer 100 may alternatively include a plurality of ventilation channels 3.

The ventilation channel 3 may include a main channel section 35 and a narrowed section 33 and/or an expanded section 34. The narrowed section 33 or the expanded section 34 may include a narrowed portion and/or an expanded portion, or the narrowed section 33 or the expanded section 34 may form a narrowed portion and/or an expanded portion together with the main channel section 35. In one embodiment, the ventilation channel 3 may include only the main channel section 35 and the narrowed section 33 that are communicated with each other. In another embodiment, the ventilation channel 3 may include only the main channel section 35 and the expanded section 34 that are communicated with each other. In another embodiment, the ventilation channel 3 may include a plurality of main channel sections 35, at least one narrowed section 33, and at least one expanded section 34 that are communicated with each other, or may be designed as needed.

In some implementations of this application, the ventilation channel 3 is defined in the mounting base 2. The ventilation channel 3 may be directly formed in the mounting base 2, or the ventilation channel 3 may be formed in a separate structural member and the separate structural member may be mounted on the mounting base 2.

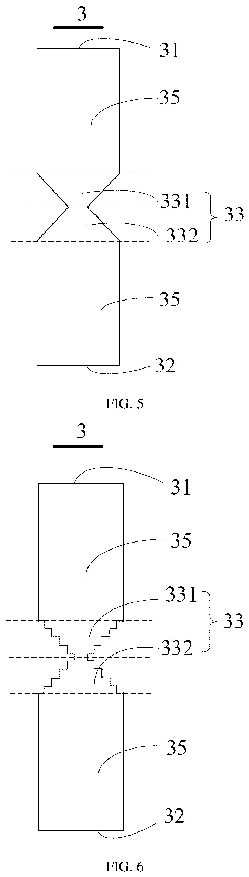

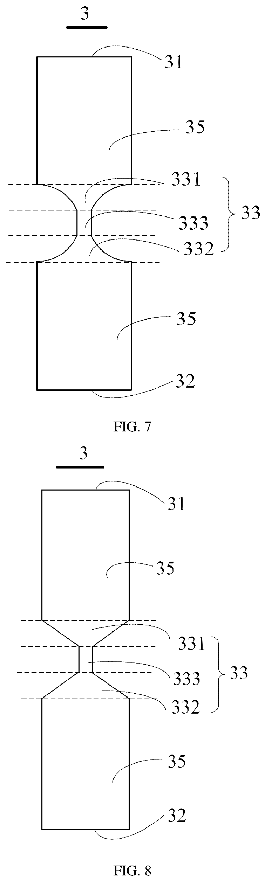

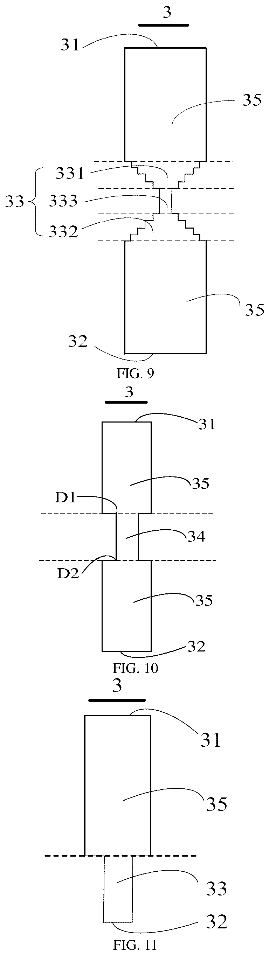

Referring to FIGS. 3-11, FIG. 3 is a schematic diagram of a structure of a first embodiment of a mounting base of the atomizer provided in FIG. 2. FIG. 4 is a schematic diagram of a structure of a first implementation of a ventilation channel of the mounting base provided in FIG. 3. FIG. 5 is a schematic diagram of a structure of a second implementation of a ventilation channel of the mounting base provided in FIG. 3. FIG. 6 is a schematic diagram of a structure of a third implementation of a ventilation channel of the mounting base provided in FIG. 3. FIG. 7 is a schematic diagram of a structure of a fourth implementation of a ventilation channel of the mounting base provided in FIG. 3. FIG. 8 is a schematic diagram of a structure of a fifth implementation of a ventilation channel of the mounting base provided in FIG. 3. FIG. 9 is a schematic diagram of a structure of a sixth implementation of a ventilation channel of the mounting base provided in FIG. 3. FIG. 10 is a schematic diagram of a structure of a seventh implementation of a ventilation channel of the mounting base provided in FIG. 3. FIG. 11 is a schematic diagram of a structure of an eighth implementation of a ventilation channel of the mounting base provided in FIG. 3.

In this embodiment, the ventilation channel 3 is directly formed in the mounting base 2. In some implementations, the atomizer 100 may include a sealing member (not shown in the figure), and the sealing member covers the end surface of the mounting base 2 close to the liquid storage cavity 13 and covers the outer side surface of the mounting base 2. The side wall of the sealing member abuts against the housing 1 and the mounting base 2 to seal the liquid storage cavity 13, so as to prevent leakage of the aerosol-forming medium in the liquid storage cavity 13. Referring to FIG. 3, the outer side surface of the mounting base 2 is provided with a micro groove, and the sealing member covers the micro groove in the outer side surface of the mounting base 2 to form the ventilation channel 3. In some implementations, the sealing member and/or the mounting base 2 may be made of a lipophilic material, so that the formed ventilation channel 3 has a lipophilic property.

As shown in FIG. 3, the ventilation channel 3 may be in a shape of a straight line structure. In this embodiment, the first end 31 of the ventilation channel 3 is directly communicated with the liquid storage cavity 13, and the second end 32 is communicated with the atomization cavity 21. The ventilation channel 3 realizes the liquid storage and ventilation functions under an action of capillary force. In some implementations, the external atmosphere enters the second end 32 of the ventilation channel 3 through the atomization cavity 21, and then enters the liquid storage cavity 13 from the first end 31 through the ventilation channel 3 to implement the ventilation process. The aerosol-forming medium in the liquid storage cavity 13 enters the ventilation channel 3 from the first end 31 of the ventilation channel 3 under the action of the capillary force to implement the liquid storage function.

In other implementations, the second end 32 of the ventilation channel 3 may alternatively not be directly communicated with the atomization cavity 21. For example, the second end 32 of the ventilation channel 3 may be directly communicated with the external atmosphere, and the external atmosphere directly enters the ventilation channel 3 through the second end 32 of the ventilation channel 3 to implement the ventilation process.

In this embodiment, the ventilation channel 3 may include only the main channel section 35 and the narrowed section 33. The quantity of the main channel sections 35 and the narrowed sections 33 may be one or more. As shown in FIG. 3, the ventilation channel 3 may include two main channel sections 35 and one narrowed section 33 that are communicated with each other. The narrowed section 33 is located between the two main channel sections 35. The two main channel sections 35 are communicated with the liquid storage cavity 13 and the atomization cavity 21, respectively. In other implementations, the quantity of narrowed sections 33 may be two or more. For example, the ventilation channel 3 may include three main channel sections 35 and two narrowed sections 33. The two narrowed sections 33 are communicated with each other via one main channel section 35. The first end 31 and the second end 32 of the ventilation channel 3 are both main channel sections 35. It may be understood that when the ventilation channel 3 includes a plurality of narrowed sections 33, during the liquid storage process of the ventilation channel 3, the plurality of narrowed sections 33 may prevent the aerosol-forming medium from continuing to flow toward the second end 32. The resistance to the aerosol-forming medium is strong, and the effect is significant, so that the aerosol-forming medium in the ventilation channel 3 can be effectively prevented from flowing out from the second end 32.

In this embodiment, the narrowed portion and/or expanded portion may be directly formed at the narrowed section 33, or may be jointly formed by the narrowed section 33 and the main channel section 35.

Referring to 4 to 6, in some implementations, the narrowed portion and the expanded portion are directly formed at the narrowed section 33. In some implementation, the equivalent diameter of the narrowed section 33 first gradually decreases and then gradually increases along a direction from the first end 31 to the second end 32 of the ventilation channel 3. The narrowed section 33 includes a first narrowed portion 331 and a first expanded portion 332. The equivalent diameter of the first narrowed portion 331 gradually decreases along the direction from the first end 31 to the second end 32 of the ventilation channel 3, and the equivalent diameter of the first expanded portion 332 gradually increases along the direction from the first end 31 to the second end 32 of the ventilation channel 3. In other words, the equivalent diameter at a position where the first narrowed portion 331 is connected to the first expanded portion 332 is the minimum equivalent diameter of the first narrowed portion 331 and the first expanded portion 332. The equivalent diameter of the main channel section 35 remains unchanged along the direction from the first end 31 to the second end 32 of the ventilation channel 3, and the equivalent diameter at a position where the first narrowed portion 331 is connected to the main channel section 35 and the equivalent diameter at a position where the first expanded portion 332 is connected to the main channel section 35 are the maximum equivalent diameter of the first narrowed portion 331 and the first expanded portion 332.

The maximum equivalent diameter of the narrowed section 33 is 0.2 mm to 1 mm, and the minimum equivalent diameter of the narrowed section 33 is shorter than the maximum equivalent diameter of the narrowed section 33. The ratio of the maximum equivalent diameter to the minimum equivalent diameter of the narrowed section 33 is M. In some implementations, 1<M<4. It may be understood that, if the maximum equivalent diameter of the narrowed section 33 is excessively long, the sum of the maximum along-the-way resistance and capillary force is not sufficient to prevent the aerosol-forming medium from flowing out of the ventilation channel 3, and leakage of the aerosol-forming medium in the ventilation channel 3 is prone to occurrence.

The micro groove in the mounting base 2 may be in any shape, to form ventilation channels 3 of different shapes. For example, the cross-sectional shape of the ventilation channel 3 may be of any shape such as a circle, a rectangle, a square, or a triangle. The narrowed section 33 of the ventilation channel 3 may transition in any form along the direction from the first end 31 to the second end 32 of the ventilation channel 3.

As shown in FIG. 4, in some implementations, the narrowed section 33 of the ventilation channel 3 transitions in a curved line form along the direction from the first end 31 to the second end 32 of the ventilation channel 3, and the side wall of the micro groove, which is provided in the outer side surface of the mounting base 2, at positions corresponding to the first narrowed portion 331 and the first expanded portion 332 is curved. In some implementations, the side wall of the micro groove at the positions corresponding to the first narrowed portion 331 and the first expanded portion 332 is arcuate, and the side edges of the first narrowed portion 331 and the first expanded portion 332 on the longitudinal section of the narrowed section 33 are in a shape of arc lines.

As shown in FIG. 5, in other implementations, the narrowed section 33 of the ventilation channel 3 transitions in a straight line form along a direction from the first end 31 to the second end 32 of the ventilation channel 3, the side wall of the micro groove, which is provided on the outer side surface of the mounting base 2, at positions corresponding to the first narrowed portion 331 and the first expanded portion 332 is planar, the side edges of the first narrowed portion 331 and the first expanded portion 332 on the longitudinal section of the narrowed section 33 are straight lines, and the side edges of the narrowed section 33 are in a shape of V.

As shown in FIG. 6, in other implementations, the narrowed section 33 of the ventilation channel 3 transitions in a stepwise form along a direction from the first end 31 to the second end 32 of the ventilation channel 3, and the side wall of the micro groove, which is provided on the outer side surface of the mounting base 2, at positions corresponding to the first narrowed portion 331 and the first expanded portion 332 is folded, and the side edges of the first narrowed portion 331 and the first expanded portion 332 on the longitudinal section of the narrowed section 33 are in a shape of stepwise lines.

In some implementations, the narrowed section 33 may include a first uniform connection portion 333. The first uniform connection portion 333 is connected between the first narrowed portion 331 and the first expanded portion 332. The equivalent diameter of the first uniform connection portion 333 remains unchanged along the direction from the first end 31 to the second end 32 of the ventilation channel 3. The equivalent diameter of the first uniform connection portion 333 is the minimum equivalent diameter of the first narrowed portion 331 and the first expanded portion 332. The cross-sectional shape of the first uniform connection portion 333 may be any shape, for example, a rectangle, a circle, or a square.

As shown in FIG. 7, in some implementations, the narrowed section 33 may include a first uniform connection portion 333. The first uniform connection portion 333 is connected between the first narrowed portion 331 and the first expanded portion 332. The first narrowed portion 331 and the first expanded portion 332 have same shapes as the first narrowed portion 331 and the first expanded portion 332 in the narrowed section 33 shown in FIG. 4, and both transition in a curved line form along the direction from the first end 31 to the second end 32 of the ventilation channel 3.

As shown in FIG. 8, in other implementations, the narrowed section 33 may include a first uniform connection portion 333. The first uniform connection portion 333 is connected between the first narrowed portion 331 and the first expanded portion 332. The shapes of the first narrowed portion 331 and the first expanded portion 332 have same shapes as the first narrowed portion 331 and the first expanded portion 332 in the narrowed section 33 shown in FIG. 5, and both transition in a straight line form along the direction from the first end 31 to the second end 32 of the ventilation channel 3.

As shown in FIG. 9, in other implementations, the narrowed section 33 may include a first uniform connection portion 333 communicating the first narrowed portion 331 and the first expanded portion 332. The shapes of the first narrowed portion 331 and the first expanded portion 332 have same shapes as the first narrowed portion 331 and the first expanded portion 332 in the narrowed section 33 shown in FIG. 6, and both transition in a stepwise form along the direction from the first end 31 to the second end 32 of the ventilation channel 3.

It may be understood that in other implementations, the narrowed section 33 may alternatively include only the first narrowed portion 331, but not the first expanded portion 332. The first narrowed portion 331 may transition in any form such as a straight line transition form, a curved line transition form, or a stepwise line transition form along the direction from the first end 31 to the second end 32.

Referring to 10 and 11, in other implementations, the narrowed portion and/or expanded portion are/is jointly formed by the narrowed section 33 and the main channel section 35.

As shown in FIG. 10, in some implementations, the ventilation channel 3 may include two main channel sections 35 and one narrowed section 33. The narrowed section 33 is located between the two main channel sections 35. Sections at the first end 31 and the second end 32 are both the main channel sections 35. In this implementation, the narrowed section 33 does not include the first narrowed portion 331 and the first expanded portion 332, and the equivalent diameter of the narrowed section 33 remains unchanged along a direction from the first end 31 to the second end 32 of the ventilation channel 3, and the equivalent diameter of the narrowed section 33 is shorter than the equivalent diameter of the main channel section 35. Because the equivalent diameter of the narrowed section 33 is shorter than the equivalent diameter of the main channel section 35, along the direction from the first end 31 to the second end 32, at a first connection position D1 between the main channel section 35 and the narrowed section 33, the ventilation channel 3 transitions from the main channel section 35 to the narrowed section 33, and the equivalent diameter of the ventilation channel 3 abruptly decreases to form a narrowed portion. At a second connection position D2 between the main channel section 35 and the narrowed section 33, the ventilation channel 3 transitions from the narrowed section 33 to the main channel section 35, and the equivalent diameter of the ventilation channel 3 abruptly increases to form an expanded portion, so that during a liquid storage process, the capillary force direction A in the ventilation channel 3 is reversed at the expanded portion, to prevent the aerosol-forming medium from flowing out of the ventilation channel 3 from the second end 32 of the ventilation channel 3.

As shown in FIG. 11, in other implementations, the ventilation channel 3 may include only one main channel section 35 and one narrowed section 33, the main channel section 35 is at the first end 31, the narrowed section 33 is at the second end 32, and the equivalent diameter of the main channel section 35 is longer than the equivalent diameter of the narrowed section 33. The narrowed section 33 does not include the first narrowed portion 331 and the first expanded portion 332, and the equivalent diameter of the narrowed section 33 remains unchanged along a direction from the first end 31 to the second end 32 of the ventilation channel 3. Along the direction from the first end 31 to the second end 32, the ventilation channel 3 transitions from the main channel section 35 to the narrowed section 33. At a connection position between the main channel section 35 and the narrowed section 33 of the ventilation channel 3, the equivalent diameter of the ventilation channel 3 abruptly decreases to form a narrowed portion.

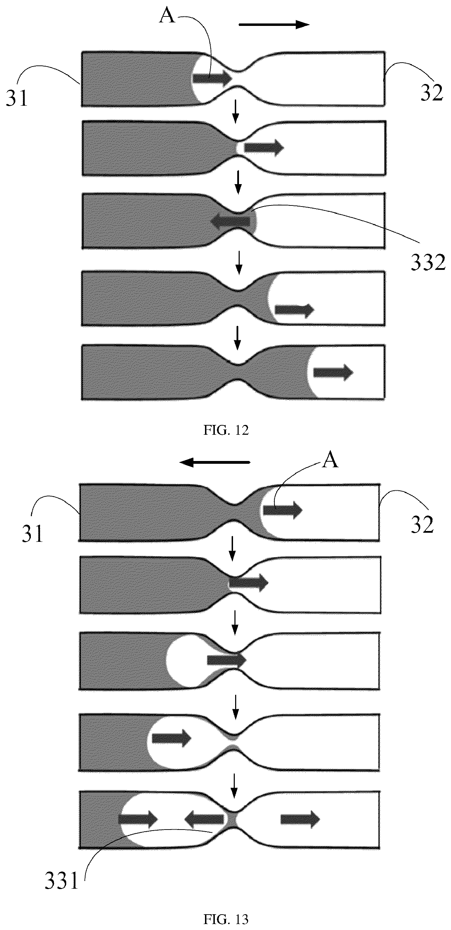

Referring to FIGS. 12-13, FIG. 12 is a schematic diagram of a structure of the ventilation channel shown in FIG. 4 during a liquid storage process, and FIG. 13 is a schematic diagram of a structure of the ventilation channel shown in FIG. 4 during a ventilation process.

In this embodiment, the side wall of the ventilation channel 3 has a lipophilic property, the first end 31 of the ventilation channel 3 is communicated with the liquid storage cavity 13, and the second end 32 is communicated with the atomization cavity 21. The narrowed section 33 is provided in the ventilation channel 3, and the capillary force direction A in the ventilation channel 3 changes at the first expanded portion 332, so that a problem that the aerosol-forming medium in the ventilation channel 3 flows out of the ventilation channel 3 from the second end 32 and then flows out of the atomizer 100 can be effectively prevented.

In some implementations, as shown in FIG. 12, when the pressure in the liquid storage cavity 13 increases, the liquid storage process is performed in the ventilation channel 3, and the aerosol-forming medium in the ventilation channel 3 flows from the first end 31 to the second end 32, in other words, the aerosol-forming medium flows from the side where the liquid storage cavity 13 is located to the side where the atomization cavity 21 is located. In a process of the aerosol-forming medium flowing from the first end 31 to the second end 32, because the side wall of the ventilation channel 3 has a lipophilic property, at the main channel section 35 corresponding to the first end 31 of the ventilation channel 3, the capillary force direction A in the ventilation channel 3 points to the side where the atomization cavity 21 is located, and at the first expanded portion 332 of the narrowed section 33, the capillary force direction A in the ventilation channel 3 changes, and the capillary force direction A originally pointing to the side where the atomization cavity 21 is located changes to pointing to the side where the liquid storage cavity 13 is located. In other words, the original capillary force direction A points from the first end 31 to the second end 32, and when aerosol-forming medium passes through the first expanded portion 332, the capillary force direction A points from the second end 32 to the first end 31. The capillary force direction A at the first expanded portion 332 is opposite to the capillary force direction A at other positions. The capillary force pointing to the side where the liquid storage cavity 13 is located prevents the aerosol-forming medium in the ventilation channel 3 from continuing to flow toward the side where the atomization cavity 21 is located, to prevent a problem that the aerosol-forming medium in the ventilation channel 3 flows out of the ventilation channel 3 from the second end 32 of the ventilation channel 3 and then flows out of the atomizer 100.

As shown in FIG. 13, when the pressure in the liquid storage cavity 13 decreases, the ventilation process is performed in the ventilation channel 3 to realize the ventilation function. To be specific, during a process of the air flowing from the second end 32 to the first end 31 of the ventilation channel 3 to ventilate the liquid storage cavity 13, the aerosol-forming medium stored in the ventilation channel 3 flows from the second end 32 to the first end 31 and returns to the liquid storage cavity 13. Because the side wall of the ventilation channel 3 has a lipophilic property, before the aerosol-forming medium flows through the first narrowed portion 331, the capillary force direction A points to the side where the atomization cavity 21 is located, in other words, the capillary force direction A points from the first end 31 to the second end 32. When the aerosol-forming medium passes through the first narrowed portion 331, the capillary force direction A changes, the air in the ventilation channel 3 flows to the first narrowed portion 331, and at the same time, the residual aerosol-forming medium in the ventilation channel 3 forms a thin liquid film at the first narrowed portion 331. Subsequently, an air-liquid mixing phenomenon occurs in the ventilation channel 3 when the air breaks through the thin liquid film, to complete the ventilation process.

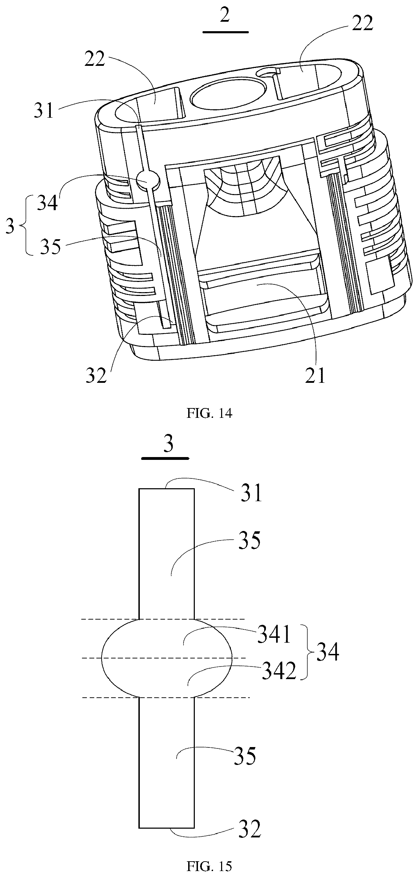

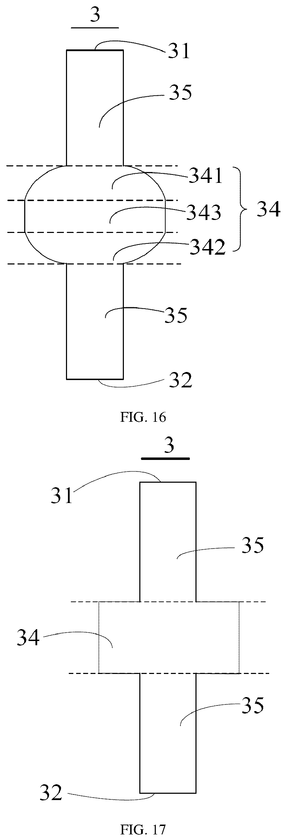

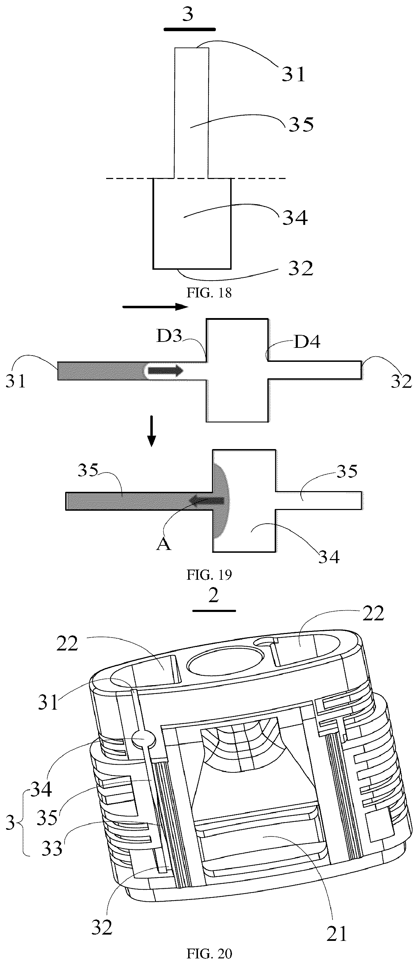

Referring to FIGS. 14-19, FIG. 14 is a schematic diagram of a structure of a second embodiment of a mounting base of the atomizer provided in FIG. 2; FIG. 15 is a schematic diagram of a structure of a first implementation of a ventilation channel of the mounting base provided in FIG. 14. FIG. 16 is a schematic diagram of a structure of a second implementation of a ventilation channel of the mounting base provided in FIG. 14. FIG. 17 is a schematic diagram of a structure of a third implementation of a ventilation channel of the mounting base provided in FIG. 14. FIG. 18 is a schematic diagram of a structure of a fourth implementation of a ventilation channel of the mounting base provided in FIG. 14. FIG. 19 is a schematic diagram of a structure of the ventilation channel shown in FIG. 17 during a liquid storage process.

Referring to FIG. 14, the ventilation channel 3 in this embodiment is formed in the same manner as the ventilation channel 3 in the first embodiment of the mounting base 2, both of which are formed by covering a micro groove in the mounting base 2 with a sealing member. In some implementations, the sealing member and/or the mounting base 2 may be made of a lipophilic material, so that the formed ventilation channel 3 has a lipophilic property.

The mounting base 2 in this embodiment is different from the first embodiment of the mounting base 2 in that a structure of the ventilation channel 3 on the mounting base 2 in this embodiment is different from the ventilation channel 3 in the first embodiment of the mounting base 2, and the remaining structures are the same as those in the first embodiment of the mounting base 2. Details are not described herein again.

In some implementations, the ventilation channel 3 may include a plurality of main channel sections 35 and at least one expanded section 34. As shown in FIG. 14, the ventilation channel 3 includes two main channel sections 35 and one expanded section 34 communicated with each other. The expanded section 34 is located between the two main channel sections 35. The two main channel sections 35 are communicated with the liquid storage cavity 13 and the atomization cavity 21, respectively. In other implementations, the quantity of expanded sections 34 may be two or more. For example, the ventilation channel 3 may include three main channel sections 35 and two expanded sections 34. The two expanded sections 34 are communicated through one main channel section 35. The first end 31 and the second end 32 of the ventilation channel 3 are both main channel sections 35. It may be understood that, when the ventilation channel 3 includes a plurality of expanded sections 34, during the liquid storage process in the ventilation channel 3, the plurality of expanded sections 34 may prevent the aerosol-forming medium from continuing to flow toward the second end 32. The resistance to the aerosol-forming medium is strong, and the effect is significant. **In** addition, the liquid storage volume in the ventilation channel 3 is large, so that the aerosol-forming medium in the ventilation channel 3 can be effectively prevented from flowing out from the second end 32.

In this embodiment, the narrowed portion and/or expanded portion may be directly formed at the expanded section 34, or may be jointly formed by the expanded section 34 and the main channel section 35.

Refer to FIG. 15, in some implementations, the narrowed portion and the expanded portion are directly formed at the expanded section 34. In some implementations, the equivalent diameter of the expanded section 34 may first gradually increase and then gradually decrease along a direction from the first end 31 to the second end 32 of the ventilation channel 3. The expanded section 34 includes a second expanded portion 341 and a second narrowed portion 342. The equivalent diameter of the second expanded portion 341 gradually increases along the direction from the first end 31 to the second end 32 of the ventilation channel 3, and the equivalent diameter of the second narrowed portion 342 gradually decreases along the direction from the first end 31 to the second end 32 of the ventilation channel 3. In other words, the equivalent diameter at a position where the second expanded portion 341 is connected to the second narrowed portion 342 in the ventilation channel 3 is the maximum equivalent diameter of the second expanded portion 341 and the second narrowed portion 342, and the equivalent diameter of the expanded section 34 is longer than the equivalent diameter of the main channel section 35. The expanded section 34 may transition in any form such as a straight line transition form, a curved line transition form, or a stepwise line transition form along the direction from the first end 31 to the second end 32 of the ventilation channel 3.

As shown in FIG. 15, in some implementations, the expanded section 34 transitions in a curved line form along the direction from the first end 31 to the second end 32 of the ventilation channel 3, and the side wall of the micro groove, which is provided on the outer side surface of the mounting base 2, at positions corresponding to the second narrowed portion 342 and the second expanded portion 341 is curved. In some implementations, the side wall of the micro groove at the positions corresponding to the second narrowed portion 342 and the second expanded portion 341 are arcuate, and the side edges of the second narrowed portion 342 and the second expanded portion 341 on the longitudinal section of the expanded section 34 are in a shape of arc lines. In other implementations, the expanded section 34 may alternatively use a straight line transition form or a stepwise lines transition form. The straight line transition form and the stepwise lines transition form are similar to the straight line transition form and the stepwise lines transition form of the narrowed section 33 in the first embodiment of the mounting base 2. Details are not described herein again.

In some implementations, a second uniform connection portion 343 may be connected between the second expanded portion 341 and the second narrowed portion 342. The equivalent diameter of the second uniform connection portion 343 remains unchanged along the direction from the first end 31 to the second end 32 of the ventilation channel 3, and the equivalent diameter of the second uniform connection portion 343 is the maximum equivalent diameter of the second expanded portion 341 and the second narrowed portion 342. The cross-sectional shape of the second uniform connection portion 343 may be any shape such as a rectangle, a circle, or a square.

As shown in FIG. 16, the expanded section 34 may include the second uniform connection portion 343. The second uniform connection portion 343 is communicating the second expanded portion 341 and the second narrowed portion 342. The shapes of the second expanded portion 341 and the second narrowed portion 342 are the same as the shapes of the second expanded portion 341 and the second narrowed portion 342 in the expanded section 34 shown in FIG. 14, and both transition in a curved line form along the direction from the first end 31 to the second end 32 of the ventilation channel 3.

It may be understood that in other implementations, the expanded section 34 may alternatively include only the second expanded portion 341 but not the second narrowed portion 342. The second expanded portion 341 may transition in any form such as a straight line transition form, a curved line transition form, or a stepwise line transition form along the direction from the first end 31 to the second end 32.

In this implementation, the capillary force direction A in the ventilation channel 3 is reversed at the expanded section 34. During the liquid storage process, the capillary force direction A is reversed at the second expanded portion 341 of the expanded section 34, to prevent the aerosol-forming medium in the ventilation channel 3 from continuing to flow toward the side where the atomization cavity 21 is located, so as to prevent leakage of the aerosol-forming medium caused by the aerosol-forming medium in the ventilation channel 3 flowing out of the ventilation channel 3 from the second end 32.

Referring to 17 and 18, in other implementations, the narrowed portion and/or expanded portion are/is jointly formed by the expanded section 34 and the main channel section 35.

In some implementations, as shown in FIG. 17, in some implementations, the ventilation channel 3 may include two main channel sections 35 and one expanded section 34. The expanded section 34 is located between the two main channel sections 35. Sections at the first end 31 and the second end 32 are both the main channel sections 35. The equivalent diameter of the expanded section 34 may remain unchanged along the direction from the first end 31 to the second end 32 of the ventilation channel 3. To be specific, the expanded section 34 does not include the second expanded portion 341 and the second narrowed portion 342, and the equivalent diameter of the expanded section 34 is longer than the equivalent diameter of the main channel section 35. As shown in FIG. 17, the longitudinal section of the expanded section 34 is rectangular. To be specific, the shape of the side wall of the micro groove at the expanded section 34 on the mounting base 2 is a rectangular plane, and the equivalent diameter of the micro groove at the expanded section 34 is longer than the equivalent diameter at other positions of the micro groove.

Because the equivalent diameter of the expanded section 34 is longer than the equivalent diameter of the main channel section 35, along the direction from the first end 31 to the second end 32, at a third connection position D3 between the main channel section 35 and the expanded section 34, the ventilation channel 3 transitions from the main channel section 35 to the expanded section 34, and the equivalent diameter of the ventilation channel 3 abruptly increases to form an expanded portion. At a fourth connection position D4 between the main channel section 35 and the expanded section 34, the ventilation channel 3 transitions from the expanded section 34 to the main channel section 35, and the equivalent diameter of the ventilation channel 3 abruptly decreases to form a narrowed portion, so that during a liquid storage process, the capillary force direction A in the ventilation channel 3 is reversed at the expanded portion to prevent the aerosol-forming medium from flowing out of the ventilation channel 3 from the second end 32 of the ventilation channel 3.

As shown in FIG. 18, in other implementations, the ventilation channel 3 may include only one main channel section 35 and one expanded section 34, the main channel section 35 is at the first end 31, the expanded section 34 is at the second end 32, and the equivalent diameter of the expanded section 34 is longer than the equivalent diameter of the main channel section 35. The expanded section 34 does not include the second narrowed portion 342 and the second expanded portion 341, and the equivalent diameter of the expanded section 34 remains unchanged along a direction from the first end 31 to the second end 32 of the ventilation channel 3. Along the direction from the first end 31 to the second end 32, the ventilation channel 3 transitions from the main channel section 35 to the expanded section 34. At a connection position between the main channel section 35 and the expanded section 34 of the ventilation channel 3, the equivalent diameter of the ventilation channel 3 abruptly increases to form an expanded portion.

As shown in FIG. 19, the equivalent diameter of the expanded section 34 remains unchanged. When the pressure in the liquid storage cavity 13 increases, the liquid storage process is performed in the ventilation channel 3, and the aerosol-forming medium in the ventilation channel 3 flows from the first end 31 to the second end 32 of the ventilation channel 3. When the aerosol-forming medium flows in the main channel section 35, because the side wall of the ventilation channel 3 has a lipophilic property, the capillary force direction A in the ventilation channel 3 always points to the side where the atomization cavity 21 is located, in other words, the capillary force direction A points from the first end 31 to the second end 32, and the aerosol-forming medium is prone to filling the ventilation channel 3. When the aerosol-forming medium flows from the main channel section 35 into the expanded section 34, the capillary force direction A is reversed, and the capillary force direction A points from the second end 32 to the first end 31, in other words, the capillary force direction A points the side where the liquid storage cavity 13 is located. The capillary force prevents the aerosol-forming medium from continuing to flow toward the side where the atomization cavity 21 is located, in other words, prevents the aerosol-forming medium from flowing toward the second end 32, so as to prevents the aerosol-forming medium in the ventilation channel 3 from flowing out from the second end 32 of the ventilation channel 3, thereby preventing leakage of the aerosol-forming medium in the ventilation channel 3. **In** addition, because the equivalent diameter of the expanded section 34 is longer than that of the main channel section 35, the liquid storage volume of the ventilation channel 3 is effectively increased, so that a problem that the aerosol-forming medium fills up the ventilation channel 3 and leaks from the second end 32 of the ventilation channel 3 is prevented, thereby effectively improving the performance of the atomizer 100.

Referring to FIG. 20, FIG. 20 is a schematic diagram of a structure of a third embodiment of a mounting base of the atomizer provided in FIG. 2.

Referring to FIG. 20, the ventilation channel 3 in this embodiment is formed in the same manner as the ventilation channel 3 in the first embodiment of the mounting base 2, both of which are formed by covering a micro groove in the mounting base 2 with a sealing member. In some implementations, the sealing member and/or the mounting base 2 may be made of a lipophilic material, so that the formed ventilation channel 3 has a lipophilic property.

The mounting base 2 in this embodiment is different from the first embodiment of the mounting base 2 that a structure of the ventilation channel 3 on the mounting base 2 in this embodiment is different from the ventilation channel 3 in the first embodiment of the mounting base 2, and the remaining structures are the same as those in the first embodiment of the mounting base 2. Details are not described herein again.

In this embodiment, the ventilation channel 3 includes both the narrowed section 33 and the expanded section 34. As shown in FIG. 20, the ventilation channel 3 includes three main channel sections 35, one narrowed section 33, and one expanded section 34 that are communicated with each other. The expanded section 34 is located on the side of the narrowed section 33 close to the first end 31. The expanded section 34 and the narrowed section 33 are communicated through the main channel section 35. In other implementations, the quantity of the main channel sections 35, the narrowed sections 33, and the expanded sections 34 may alternatively be configured to be any other quantity. For example, the ventilation channel 3 may include six main channel sections 35, two narrowed sections 33, and three expanded sections 34, the main channel sections 35 are at the first end 31 and the second end 32, and the remaining narrowed sections 33 and expanded sections 34 may be distributed at any positions.

In this embodiment, a structure of the narrowed section 33 may be the structure of any implementation of the narrowed section 33 of the ventilation channel 3 in the first embodiment of the mounting base 2, and a structure of the expanded section 34 may also be the structure of any implementation of the expanded section 34 of the ventilation channel 3 in the second embodiment of the mounting base 2. It may be designed as needed, and is not limited in this application.

It may be understood that when the ventilation channel 3 includes both the narrowed section 33 and the expanded section 34, during the liquid storage process in the ventilation channel 3, both the narrowed sections 33 and the expanded section 34 may prevent the aerosol-forming medium from continuing to flow toward the second end 32. The resistance to the aerosol-forming medium is strong, and the effect is significant, so that the aerosol-forming medium in the ventilation channel 3 can be effectively prevented from flowing out from the second end 32, a related art problem of leakage of the aerosol-forming medium in the ventilation channel 3 can be effectively resolved, thereby improving the performance of the atomizer 100.

Some embodiments of this application may provide another atomizer 100. In this embodiment, the atomizer 100 may include a ventilation member. A ventilation channel 3 is defined in the ventilation member, and the ventilation member is disposed on a mounting base 2. The material of the ventilation member is a lipophilic material, so that the ventilation channel 3 has a lipophilic property.

In some implementations, the ventilation member is a hollow tube. The hollow tube is of a straight line structure and the hollow tube is disposed on the mounting base 2. The hollow tube may be directly attached to the outer surface of the mounting base 2, so that the ports at the two ends of the hollow tube are respectively communicated with a liquid storage cavity 13 and the atmosphere, or the ports at the two ends of the hollow tube are respectively communicated with a liquid storage cavity 13 and an atomization cavity 21, so that the hollow tube is used as the ventilation channel 3. In some implementations, a receiving groove may be defined on the side wall of the mounting base 2, and the hollow tube may be embedded in the receiving groove of the mounting base 2, so that the two ends of the hollow tube are directly communicated with a liquid storage cavity 13 and the atmosphere, respectively, or the two ends of the hollow tube are directly communicated with a liquid storage cavity 13 and an atomization cavity 21, respectively, so that the hollow tube is used as the ventilation channel 3. The hollow tube may be in any shape such as a hollow cylinder or a hollow prism.

In this embodiment, the hollow tube, namely, the ventilation channel 3, may include only a main channel section 35 and a narrowed section 33, or may include only a main channel section 35 and an expanded section 34, or may include a main channel section 35, a narrowed section 33, and an expanded section 34, so that the ventilation channel 3 has a narrowed portion and/or an expanded portion. In some implementations, a part of the wall of the hollow tube curves inward to form a narrowed section 33, in other words, the outer diameter and inner diameter of the hollow tube at the narrowed section 33 reduces, and/or a part of the wall of the hollow tube is expanded to form an expanded section 34, in other words, the outer diameter and inner diameter of the hollow tube at the expanded section 34 increases. Structures of the narrowed section 33 and the expanded section 34 of the hollow tube may be the same as the structures of the narrowed section 33 and the expanded section 34 in any of the foregoing implementations. Details are not described herein again. In other implementations, the ventilation member may alternatively be configured as a structural member of another shape, and the ventilation channel 3 is defined in the ventilation member.

It may be understood that the material of the ventilation member is a lipophilic material, so that the ventilation channel 3 has a lipophilic property, and a capillary force direction A in the ventilation channel 3 always points from a first end 31 to a second end 32. In this embodiment, the ventilation channel 3 is formed in the ventilation member, and the ventilation member is mounted on the mounting base 2. The ventilation channel 3 includes the narrowed section 33 and/or the expanded section 34, so that the ventilation channel 3 has a narrowed portion and/or an expanded portion. The capillary force direction A in the ventilation channel 3 is reversed at the narrowed section 33 and the expanded section 34. The capillary force direction A is reversed at the narrowed portion or the expanded portion of the narrowed section 33 and/or expanded section 34 of the ventilation channel 3, to effectively prevent a related art problem of leakage of the aerosol-forming medium caused by the aerosol-forming medium flowing out of the ventilation channel 3 from the second end 32 when the ventilation channel 3 is filled up with the aerosol-forming medium because the aerosol-forming medium in the ventilation channel 3 continuously flows from the first end 31 to the second end 32 due to the capillary force direction A in the ventilation channel 3 in the liquid storage cavity 13 pointing from the first end 31 to the second end 32.

In this embodiment, the ventilation member may be provided with only one ventilation channel 3 or may be provided with a plurality of ventilation channels 3. When the ventilation member is provided with only one ventilation channel 3, a related art problem of leakage of the aerosol-forming medium caused by the asymmetric pressure in a plurality of ventilation channels 3 when the atomizer 100 includes the plurality of ventilation channels 3 may be resolved.

Some embodiments of this application may provide another atomizer 100. In this embodiment, the atomizer 100 includes a liquid storage cavity 13 and a ventilation channel 3. The liquid storage cavity 13 is configured to store an aerosol-forming medium. A first end 31 of the ventilation channel 3 is communicated with the liquid storage cavity 13. The side wall of the ventilation channel 3 has a liquid-repellent property, for example, hydrophobicity or oleophobicity, to prevent the aerosol-forming medium from flowing out of the ventilation channel 3 from a second end 32 of the ventilation channel 3, thereby preventing a liquid leakage problem in the atomizer 100.

In other words, the atomizer 100 in this embodiment is different from the first and second embodiments of the atomizer 100 in that, in this embodiment, the aerosol-forming medium is an oily liquid, and the side wall of the ventilation channel 3 of the atomizer 100 has an oleophobic property.

In some implementations, the ventilation channel 3 may be made of an oleophobic material. For example, the side wall of the ventilation channel 3 may be directly made of an oleophobic material, for example, PDMS or Teflon. In some implementations, the side wall of the ventilation channel 3 may be formed by an oleophobic material prepared from a lipophilic material through treatment. In some implementations, the side wall of the ventilation channel 3 may be coated with an oleophobic material coating so that the side wall of the ventilation channel 3 has an oleophobic property. In some implementations, the side wall of the ventilation channel 3 may alternatively be configured to have an oleophobic micro-nano structure, so that the side wall of the ventilation channel 3 has an oleophobic property.

It may be understood that in this embodiment, the side wall of the ventilation channel 3 has an oleophobic property, so that during a flow process of the aerosol-forming medium in the ventilation channel 3, a capillary force direction A in the ventilation channel 3 always points to the side where the liquid storage cavity 13 is located. In other words, when a liquid storage process or a ventilation process is performed in the ventilation channel 3, due to the oleophobic property of the side wall of the ventilation channel 3, a capillary force direction A in the ventilation channel 3 always points from the second end 32 to the first end 31. During the liquid storage process in the ventilation channel 3, that is, during the process of the aerosol-forming medium flowing from the first end 31 to the second end 32 of the ventilation channel 3 due to increase of the pressure in the liquid storage cavity 13, when the external temperature or pressure changes, the pressure difference between the inside and outside of the liquid storage cavity 13 changes, but the capillary force in the ventilation channel 3 overcomes the pressure difference between the inside and outside, to prevent the aerosol-forming medium from continuing to flow toward the second end 32 of the ventilation channel 3, so as to prevent liquid leakage caused by the aerosol-forming medium in the ventilation channel 3 flowing out of the ventilation channel 3 from the second end 32. During a ventilation process in the ventilation channel 3, that is, during the external air and the aerosol-forming medium in the ventilation channel 3 flowing from the second end 32 to the first end 31 of the ventilation channel 3 due to the low pressure in the liquid storage cavity 13 is, the capillary force direction A always points from the second end 32 to the first end 31. Under the joint action of the pressure difference between the inside and the outside and the capillary force, the along-the-way resistance of the flow process of the aerosol-forming medium in the ventilation channel 3 is overcome, so as to facilitate ventilation and achieve a smooth ventilation process and high ventilation efficiency, thereby reducing a risk of the atomizer 100 producing a burnt taste.

In this embodiment, the remaining structures of the atomizer 100 may be the same as the structures of the first embodiment or the second embodiment of the atomizer 100. Details are not described herein again. The ventilation channel 3 may be formed in any one of the manners in the first embodiment or the second embodiment of the atomizer 100. In some implementations, the ventilation channel 3 may be directly formed in the mounting base 2. For example, the atomizer 100 includes a sealing member, and the outer side surface of the mounting base 2 is provided with a micro groove, and the sealing member covers the micro groove in the outer side surface of the mounting base 2 to form the ventilation channel 3. The material of the sealing member and/or the material of the mounting base 2 are/is an oleophobic material, so that the side wall of the ventilation channel 3 has an oleophobic property. In some implementations, the ventilation channel 3 may be formed in a separate structural member and the separate structural member may be mounted on the mounting base 2. For example, the atomizer 100 includes a ventilation member, and the ventilation member may be a hollow tube, so that the hollow tube is used as the ventilation channel 3. The material of the ventilation member is an oleophobic material, so that the side wall of the ventilation channel 3 has an oleophobic property to prevent the aerosol-forming medium in the ventilation channel 3 from flowing out of the ventilation channel 3 from the second end 32.

The structure of the ventilation channel 3 may be the same as the structure of any implementation of the ventilation channel 3 in the first embodiment or the second embodiment of the atomizer 100. In other words, in this embodiment, the ventilation channel 3 may include a narrowed portion and/or an expanded portion. Because the side wall of the ventilation channel 3 in this embodiment has an oleophobic property, the capillary force direction A in the ventilation channel 3 points from the second end 32 to the first end 31 of the ventilation channel 3, and the capillary force direction A does not change at the expanded portion. Even if the capillary force direction A changes at the narrowed portion, the impact may be ignored because the capillary force direction A at other positions in the ventilation channel 3 always points to the first end 31, so that the aerosol-forming medium can be effectively prevented from flowing out of the ventilation channel 3 from the second end 32 of the ventilation channel 3.

It may be understood that in other implementations, the ventilation channel 3 may not include the narrowed portion and the expanded portion, and the ventilation channel 3 may only include a main channel section 35. In other words, the equivalent diameter of the ventilation channel 3 may remain unchanged along a direction from the first end 31 to the second end 32, or the equivalent diameter of the ventilation channel 3 may alternatively change in any form along the direction from the first end 31 to the second end 32, or the ventilation channel 3 may be in any irregular shape, provided that the side wall of the ventilation channel 3 has an oleophobic property. In this embodiment, because the side wall of the ventilation channel 3 has an oleophobic property, even if the ventilation channel 3 does not include the narrowed portion and the expanded portion, the capillary force direction A in the ventilation channel 3 always points from the second end 32 to the first end 31 of the ventilation channel 3. The capillary force in the ventilation channel 3 prevents the aerosol-forming medium from continuing to flow toward the second end 32, so as to effectively prevent a liquid leakage problem caused by the aerosol-forming medium flowing out of the second end 32, thereby improving the performance of the atomizer 100.

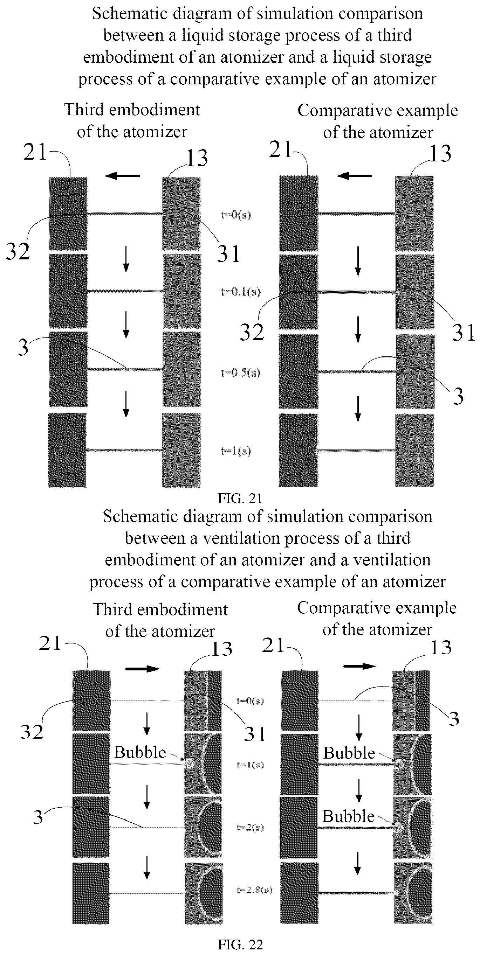

Referring to FIGS. 21-22, FIG. 21 is a schematic diagram of simulation comparison between a liquid storage process of a third embodiment of an atomizer and a liquid storage process of a comparative example of an atomizer; and FIG. 22 is a schematic diagram of simulation comparison between a ventilation process of a third embodiment of an atomizer and a ventilation process of a comparative example of an atomizer.

To verify the performance of a ventilation channel 3 of the third embodiment of the atomizer 100, that is, to verify the performance of a ventilation channel 3 having an oleophobic property, the inventor of this application respectively uses the third embodiment of the atomizer 100 and the comparison example of the atomizer to separately carry out experiments on the two different atomizers 100 under the same experimental conditions.

Specifically, the third embodiment of the atomizer 100 is only different from the comparative example of the atomizer in that the side wall of the ventilation channel 3 of the third embodiment of the atomizer 100 is made of an oleophobic material, while the side wall of the ventilation channel 3 of the comparative example of the atomizer is made of a lipophilic material. The remaining structures of the third embodiment of the atomizer 100 and the comparative example of the atomizer are exactly the same. The same experiments are carried out on the two atomizers 100, and experimental results shown in FIG. 21 and FIG. 22 are obtained.

First, the comparative experiment is carried out on the liquid storage process in the ventilation channel 3 of the third embodiment of the atomizer 100 and the liquid storage process in the ventilation channel 3 of the comparative example of the atomizer under the same conditions. The experimental simulation results are shown in FIG. 21. The liquid storage process of the third embodiment of the atomizer 100 (left side in FIG. 21) and the liquid storage process of the comparative example of the atomizer (right side in FIG. 22) start at the same time at the second of t=0. In the comparative example of the atomizer on the right, the side wall of the ventilation channel 3 is made of a lipophilic material, a capillary force direction A in the ventilation channel 3 points from a first end 31 to a second end 32, that is, a capillary force direction A points from a liquid storage cavity 13 to an atomization cavity 21/ the external atmosphere, and the ventilation channel 3 actively absorbs liquid from the liquid storage cavity 13, so that an aerosol-forming medium flows fast in the ventilation channel 3 of the lipophilic material, while in the ventilation channel 3 of the third embodiment of the atomizer 100 on the left, because the side wall of the ventilation channel 3 is made of an oleophobic material, a capillary force direction A in the ventilation channel 3 points from a second end 32 to a first end 31, and the capillary force prevents an aerosol-forming medium from flowing from the first end 31 to the second end 32, so that the aerosol-forming medium flows slowly in the ventilation channel 3 of the oleophobic material. At the second of t=1, the aerosol-forming medium in the third embodiment of the atomizer 100 on the left has not flowed to the second end 32 of the ventilation channel 3, while the aerosol-forming medium in the ventilation channel 3 of the comparative example of the atomizer on the right has already flowed out of the ventilation channel 3 from the second end 32 of the ventilation channel 3, resulting in a liquid leakage problem. After comparing and analyzing the experimental results, the applicant finds that when the ventilation channel 3 has an oleophobic property, a problem that the aerosol-forming medium from flows out of the ventilation channel 3 from the second end 32 of the ventilation channel 3 can be effectively prevented, so that a leakage problem of the atomizer 100 can be resolved.

The comparative experiment is carried out on the ventilation process in the ventilation channel 3 of the third embodiment of the atomizer 100 and the ventilation process in the ventilation channel 3 of the comparative example of the atomizer under the same conditions. The experimental simulation results are shown in FIG. 22. Under the same pressure, the ventilation process of the third embodiment of the atomizer 100 (left side in FIG. 21) and the ventilation process of the comparative example of the atomizer (right side in FIG. 22) start at the same time at the second of t=0. It can be seen from FIG. 22, in the ventilation channel 3 of the third embodiment of the atomizer 100 on the left, the ventilation process is completed at the second of t=2, while in the ventilation channel 3 of the comparative example of the atomizer on the right, the ventilation process is completed at the second of t=2.8. In other words, the ventilation process in the ventilation channel 3 of the oleophobic material is completed faster than that of the ventilation channel 3 of the lipophilic material. After analyzing and comparing the experimental results, the inventor finds that the ventilation channel 3 of the third embodiment of the atomizer 100 has an oleophobic property, the capillary force direction A points to the side where the liquid storage cavity 13 is located, that is, from the second end 32 to the first end 31, and the capillary force and the pressure difference between the inside and the outside may work together to overcome the along-the-way resistance, to achieve a smooth ventilation process in the ventilation channel 3 and high ventilation efficiency, thereby reducing a risk of the atomizer 100 producing a burnt taste and improving the atomization performance.

After comparing and analyzing the experimental results of the liquid storage processes and the ventilation processes of the two atomizers 100 having the ventilation channels 3 with different characteristics, the inventor finally verifies that during the liquid storage process, the ventilation channel 3 having an oleophobic property may effectively prevent the aerosol-forming medium from flowing toward the second end 32 of the ventilation channel 3, to resolve a related art problem of liquid leakage of the atomizer 100 caused by the aerosol-forming medium in the ventilation channel 3 continuously flowing toward the second end 32 and flowing out of the ventilation channel 3 from the second end 32 of the ventilation channel 3, thereby effectively improving the atomization performance of the atomizer 100. Moreover, during the ventilation process in the ventilation channel 3, the ventilation channel 3 having an oleophobic property may also make the capillary force and the force produced from the pressure difference between the inside and the outside act in the same direction, and the capillary force and the force produced from the pressure difference between the inside and the outside jointly overcome the along-the-way resistance when the aerosol-forming medium flows from the second end 32 to the first end 31, to facilitate ventilation and achieve a smooth ventilation process and high ventilation efficiency, so that a related art problem of ventilation difficulty in the ventilation channel is effectively resolved, thereby reducing a risk of the atomizer 100 producing a burnt taste.

Different from the related art, some embodiments of this application disclose an atomizer 100 and an electronic atomization apparatus 300. The atomizer 100 includes a liquid storage cavity 13 and a ventilation channel 3. The liquid storage cavity 13 is configured to store an aerosol-forming medium. A first end 31 of the ventilation channel 3 is communicated with the liquid storage cavity 13. The ventilation channel 3 includes a narrowed portion and/or an expanded portion, to prevent the aerosol-forming medium from flowing out of the ventilation channel 3 from a second end 32 of the ventilation channel 3. Based on the foregoing configuration, a structure of the narrowed portion and/or the expanded portion of the ventilation channel 3 changes the capillary force direction A in the ventilation channel 3, effectively preventing the aerosol-forming medium in the ventilation channel 3 from flowing out of the ventilation channel 3 from the second end 32, thereby resolving a related art problem that the aerosol-forming medium in the ventilation channel is prone to leakage and improving the performance of the atomizer 100.

The foregoing descriptions are merely some embodiments of this application, and the patent scope of this application is not limited thereto. All equivalent structure or process changes made based on the content of this specification and accompanying drawings in this application or by directly or indirectly applying this application in other related technical fields shall fall within the patent protection scope of this application.

## Claims

1. An atomizer, **characterized by** comprising:
a liquid storage cavity, configured to store an aerosol-forming medium; and
a ventilation channel, wherein a first end of the ventilation channel is communicated with the liquid storage cavity, and the ventilation channel comprises a narrowed portion and/or an expanded portion configured to prevent the aerosol-forming medium from flowing out of the ventilation channel from a second end of the ventilation channel.

2. The atomizer of claim 1, wherein the ventilation channel comprises a main channel section and at least one narrowed section that are communicated with each other, wherein each narrowed section comprises the narrowed portion and/or the expanded portion, or the each narrowed section and the main channel section jointly form the narrowed portion and/or the expanded portion; and/or
the ventilation channel comprises the main channel section and at least one expanded section that are communicated with each other, wherein each expanded section comprises the narrowed portion and/or the expanded portion, or the each narrowed section and the main channel section jointly form the narrowed portion and/or the expanded portion.

3. The atomizer of claim 2, wherein the ventilation channel comprises the at least one narrowed section, an equivalent diameter of the each narrowed section gradually decreases along a direction from the first end to the second end of the ventilation channel to form a first narrowed portion, and then gradually increases to form a first expanded portion, or the equivalent diameter of the each narrowed section remains unchanged along the direction from the first end to the second end of the ventilation channel, and the equivalent diameter of the each narrowed section is shorter than an equivalent diameter of the main channel section; or
the ventilation channel comprises the at least one expanded section, an equivalent diameter of the each expanded section gradually increases along a direction from the first end to the second end of the ventilation channel to form a second expanded portion, and then gradually decreases to form a second narrowed portion, or the equivalent diameter of the each expanded section remains unchanged along the direction from the first end to the second end of the ventilation channel, and the equivalent diameter of the each expanded section is longer than the equivalent diameter of the main channel section.

4. The atomizer of claim 3, wherein the ventilation channel comprises the at least one narrowed section, the each narrowed section further comprises a first uniform connection portion communicating the first narrowed portion and the first expanded portion, and an equivalent diameter of the first uniform connection portion remains unchanged along the direction from the first end to the second end of the ventilation channel; or
the ventilation channel comprises the at least one expanded section, the each expanded section further comprises a second uniform connection portion communicating the second narrowed portion and the second expanded portion, and an equivalent diameter of the second uniform connection portion remains unchanged along the direction from the first end to the second end of the ventilation channel.

5. The atomizer of claim 2, wherein the ventilation channel comprises the main channel section and the at least one narrowed section that are communicated with each other, wherein the maximum equivalent diameter of the each narrowed section is 0.2 mm to 1 mm.

6. The atomizer of claim 2, wherein the ventilation channel comprises the main channel section and the at least one narrowed section that are communicated with each other, wherein the minimum equivalent diameter of the each narrowed section is shorter than the maximum equivalent diameter of the each narrowed section; and the ratio of the maximum equivalent diameter of the each narrowed section to the minimum equivalent diameter of the each narrowed section is M, wherein 1<M<4.

7. The atomizer of claim 1, wherein the narrowed portion or the expanded portion transitions in a curved line form along a direction from the first end to the second end of the ventilation channel; or
the narrowed portion or the expanded portion transitions in a straight line form along the direction from the first end to the second end of the ventilation channel; or
the narrowed portion or the expanded portion transitions in a stepwise line form along the direction from the first end to the second end of the ventilation channel.

8. The atomizer of claim 1, wherein a side wall of the ventilation channel has a lyophilic property.

9. The atomizer of claim 8, wherein a material of the side wall of the ventilation channel is a lipophilic material; or
the side wall of the ventilation channel is provided with a lipophilic material coating; or
the side wall of the ventilation channel has a lipophilic micro-nano structure.

10. The atomizer of claim 1, wherein the atomizer comprises:
a housing, provided with an air outlet tube and an accommodating cavity; and
a mounting base, disposed in the accommodating cavity, wherein the mounting base and the housing jointly form the liquid storage cavity, the mounting base is internally provided with an atomization cavity, and the atomization cavity is communicated with the air outlet tube and external atmosphere; and
the ventilation channel is provided in the mounting base, and the second end of the ventilation channel is communicated with the atomization cavity or the external atmosphere.

11. The atomizer of claim 10, wherein the ventilation channel is in a shape of a straight line structure; and
the first end of the ventilation channel is directly communicated with the liquid storage cavity, and the second end is communicated with the atomization cavity.

12. The atomizer of claim 10, wherein the atomizer further comprises a sealing member, an outer side surface of the mounting base is provided with a micro groove, and the sealing member covers the micro groove to form the ventilation channel; or
the atomizer further comprises a ventilation member, the ventilation channel is provided in the ventilation member, and the ventilation member is disposed on the mounting base.

13. The atomizer of claim 12, wherein the atomizer comprises the ventilation member, and the ventilation member is a hollow tube used as the ventilation channel; a part of a wall of the hollow tube curves inward to form a narrowed section, and the narrowed section comprises a first narrowed portion and a first expanded portion, and/or a part of the wall of the hollow tube is expanded outward to form an expanded section, and the expanded section comprises a second narrowed portion and a second expanded portion.

14. The atomizer of claim 12, wherein a material of the sealing member and/or a material of the mounting base are/is a lipophilic material; or
a material of the ventilation member is a lipophilic material.

15. The atomizer of claim 10, wherein the atomizer further comprises an atomization core, the atomization core is disposed in the mounting base and is fluidly communicated with the liquid storage cavity, and the atomization core comprises a porous liquid-guiding substrate and a heating element.

16. The atomizer of claim 1, wherein the atomizer comprises only one ventilation channel.

17. An electronic atomization apparatus, **characterized by** comprising:
an atomizer, wherein the atomizer is the atomizer of any one of claims 1 to 16; and
a power supply assembly, electrically connected to the atomizer, and configured to provide energy to the atomizer.
